# EUROPÄISCHE PATENTANMELDUNG

(11) **EP 3 511 338 A2**
(43) Veröffentlichungstag der Anmeldung: **17.07.2019**
(21) Anmeldenummer: 18212034.5
(22) Anmeldetag: 26.04.2006
(51) Int. Cl.: C07K 14/195, C07K 14/33, A61K 38/00

(54) **CARRIER ZUM TARGETING VON NERVENZELLEN**

(30) Priorität: 26.04.2005 DE 102005019302
(62) Teilanmeldung aus: 17154062.8
(71) Anmelder: Ipsen Bioinnovation Limited, Abingdon, Oxfordshire OX14 4RY (GB)
(72) Erfinder: RUMMEL, Andreas, 30171 Hannover (DE); WEIL, Tanja, 70186 Stuttgart (DE); GUTCAITS, Aleksandrs, 1006 Riga (LV)
(74) Vertreter: Gregson, Anna Louise

(57) **Zusammenfassung**

Die vorliegende Erfindung betrifft ein Transportprotein, erhältlich durch Modifizierung der schweren Kette des von *Clostridium botulinum* gebildeten Neurotoxins, wobei (i) das Protein an Nervenzellen mit höherer oder niedrigerer Affinität bindet als das native Neurotoxin; (ii) das Protein im Vergleich zu dem nativen Neurotoxin eine erhöhte oder verringerte Neurotoxizität aufweist; vorzugsweise wird die Neurotoxizität im Hemidiaphragma-Assay bestimmt; und/oder (iii) das Protein im Vergleich zu dem nativen Neurotoxin eine geringere Affinität gegenüber neutralisierenden Antikörpern aufweist. Ferner werden Verfahren zu dessen Herstellung und dessen Verwendung in kosmetischen und pharmazeutischen Zusammensetzungen angegeben.

SEQUENCE LISTING

<110>  Ipsen Bioinnovation Limited

<120>  Carrier zum Targeting von Nervenzellen

<130>  P40113EP-D3-PCT

<150>  DE 20051019302
<151>  2005-04-26

<160>  1

<170>  PatentIn version 3.5

<210>  1
<211>  20
<212>  PRT
<213>  Artificial Sequence

<220>
<223>  cleavage site



<220>
<221>  misc_feature
<222>  (2)..(4)
<223>  Xaa can be any naturally occurring amino acid

<220>
<221>  misc_feature
<222>  (5)..(5)
<223>  Xaa can be selected from Alanine, Valine, Serine, Threonine and
Glycine

<220>
<221>  misc_feature
<222>  (17)..(17)
<223>  Xaa can be selected from Alanine, Valine, Serine, Threonine and
Glycine

<220>
<221>  misc_feature
<222>  (18)..(19)
<223>  Xaa can be any naturally occurring amino acid

<400>  1

Cys Xaa Xaa Xaa Xaa Lys Thr Lys Ser Leu Val Pro Arg Gly Ser Lys
1               5                   10                  15


Xaa Xaa Xaa Cys
            20

## Beschreibung

Die vorliegende Erfindung bezieht sich auf ein Transportprotein, das an Neurone mit höherer oder niedriger Affinität bindet als das von *Clostridium botulinum* gebildete Neurotoxin. Das Transportprotein wird vorzugsweise durch rezeptorvermittelte Endozytose aufgenommen. Dieses Protein findet Verwendung als Transporter, der andere chemische Substanzen (z.B. Proteasen) aus dem sauren, endosomalen Kompartiment in das Zytosol von Neuronen transloziert, die physiologisch nicht durch die Plasmamembran in das Zytosol von Nervenzellen eindringen können. Die vorliegende Erfindung bezieht sich insbesondere auf die Verwendung eines Transportproteins zur Einschleusung von Inhibitoren der Neurotransmitterausschüttung.

Nervenzellen setzen Transmitterstoffe durch Exozytose frei. Als Exozytose wird die Verschmelzung der Membranen intrazellulärer Vesikel mit der Plasmamembran bezeichnet. Bei diesem Vorgang wird gleichzeitig der vesikuläre Inhalt in den synaptischen Spalt ausgeschüttet. Die Fusion der beiden Membranen wird durch Calcium reguliert, welches mit dem Protein Synaptotagmin reagiert. Zusammen mit anderen Kofaktoren kontrolliert Synaptotagmin den Status von drei sogenannten Fusionsproteinen, dem SNAP-25, Synaptobrevin 2 und Syntaxin 1A. Während Syntaxin 1A und Synaptobrevin 2 in die Plasma- bzw. Vesikelmembran integriert sind, ist SNAP-25 nur schwach an die Plasmamembran gebunden. Bei einem Anstieg der intrazellulären Calcium-Konzentration binden die drei Proteine aneinander, wobei sich beide Membranen annähern und anschließend miteinander verschmelzen. Bei cholinergen Neuronen wird Acetylcholin freigesetzt, welches Muskelkontraktionen, Schweißabsonderung und andere cholinerg vermittelte Reaktionen verursacht.

Die oben genannten Fusionsproteine sind die Zielmoleküle (Substrate) der leichten Kette (LC) der clostridiellen Neurotoxine, die von den Bakterien *C. botulinum, C. butyricum, C. baratii* und *C*. *tetani* gebildet werden.

Das anaerobe, gram-positive Bakterium *C*. *botulinum* produziert sieben verschiedene Serotypen der clostridiellen Neurotoxine. Diese werden als die Botulinus Neurotoxine (BoNT/A bis BoNT/G) bezeichnet. Hiervon verursachen insbesondere BoNT/A und BoNT/B bei Mensch und Tier eine neuroparalytische Erkrankung, die als Botulismus bezeichnet wird. Die Sporen von *C*. *botulinum* finden sich im Erdreich, können sich jedoch in unsachgemäß sterilisierten und verschlossenen Nahrungsmittelkonserven aus häuslicher Herstellung entwickeln, auf die viele der Botulismusfälle zurückgeführt werden.

BoNT/A ist die aktivste aller bekannten biologischen Substanzen. Nur etwa 5-6 pg gereinigtes BoNT/A repräsentieren eine MLD (Median letale Dosis). Eine Einheit (engl.: Unit, U) von BoNT/A wird als die MLD definiert, die nach intraperitonealer Injektion die Hälfte an weiblichen Swiss Webster-Mäusen im Gewicht von jeweils 18-20 g tötet. Sieben immunologisch unterschiedliche BoNT wurden charakterisiert. Sie tragen die Bezeichnungen BoNT/A, B, Cl, D, E, F und G und können durch Neutralisation mit Serotyp spezifischen Antikörpern unterschieden werden. Die verschiedenen Serotypen von BoNT unterscheiden sich bei betroffenen Tierarten hinsichtlich der Schwere und Dauer der verursachten Lähmung. So ist z. B. bei der Ratte im Hinblick auf die Lähmung BoNT/A 500mal stärker wirksam als BoNT/B. Hinzu kommt, dass BoNT/B sich bei Primaten in einer Dosierung von 480 U/kg Körpergewicht als untoxisch erwiesen hat. Dieselbe Menge BoNT/A entspricht der 12fachen letalen Dosis dieses Stoffes bei Primaten. Zum anderen ist bei Mäusen die Lähmungsdauer nach Injektion von BoNT/A 10fach länger als nach Injektion von BoNT/E.

Die BoNT werden zur Behandlung neuromuskulärer Störungen eingesetzt, die durch Hyperaktivität in Skelettmuskeln, verursacht durch pathologisch überaktive periphere Nerven, charakterisiert sind. BoNT/A ist von der U.S. Food and Drug Administration zur Behandlung von Blepharospasmus, Strabismus, Hyperhydrose, Falten und Hemifacialspasmen zugelassen. Verglichen mit BoNT/A besitzen die übrigen BoNT Serotypen offensichtlich eine geringere Wirksamkeit und kürzere Wirkdauer. Klinische Effekte des peripher intramuskulär verabreichten BoNT/A stellen sich für gewöhnlich innerhalb einer Woche ein. Die Dauer der Symptomunterdrückung durch eine einzige intramuskuläre Injektion von BoNT/A beläuft sich im Regelfall auf etwa drei bis sechs Monate.

Die clostridiellen Neurotoxine hydrolysieren spezifisch verschiedene Proteine des Fusionsapparates. BoNT/A, C1 und E spalten SNAP-25, während BoNT/B, D, F, G sowie Tetanus Neurotoxin (TeNT) das vesikel-assoziierte Membranprotein (VAMP) 2 - auch Synaptobrevin 2 genannt - angreifen. BoNT/Cl spaltet außerdem Syntaxin 1A.

Die Clostridien Bakterien setzen die Neurotoxine als einkettige Polypeptide mit jeweils 1251 bis 1315 Aminosäuren frei. Nachfolgend spalten endogene Proteasen jedes dieser Proteine an einer bestimmten Stelle in jeweils 2 Ketten (,nicking'), wobei die beiden Ketten jedoch durch eine Disulfid-Brücke miteinander verbunden bleiben. Diese zweikettigen Proteine werden als Holotoxine bezeichnet (siehe Shone et al. (1985), Eur. J. Biochem. 151, 75-82). Die beiden Ketten haben verschiedene Funktionen. Während das kleinere Teilstück, die leichte kette (light chain = LC), eine Zn²⁺-abhängige Endoprotease darstellt, ist die größere Einheit (heavy chain = HC) der Transporter der leichten Kette. Durch Behandlung der HC mit Endopeptidasen ergaben sich zwei 50 kDa Fragmente (siehe Gimenez et al. (1993), J. Protein Chem. 12, 351-363). Die amino-terminale Hälfte (H_{N}-Fragment) integriert sich bei niedrigem pH-Wert in Membranen und transloziert die LC in das Zytosol der Nervenzelle. Die carboxyl-terminale Hälfte (H_{C}-Fragment) bindet an komplexe Polysialoganglioside, die nur in Nervenzellmembranen vorkommen, und an bislang nur teilweise identifizierte Proteinrezeptoren (Halpern et al. (1993), Curr Top Microbiol Immunol 195, 221-241). Dies erklärt die hohe Neuroselektivität der clostridiellen Neurotoxine. Kristallstrukturen bestätigen, dass BoNT/A über drei Domänen verfügt, die mit den drei Schritten des Wirkmechanismus in Einklang gebracht werden können (siehe Lacy et al. (1998), Nat. Struct. Biol. 5, 898-902). Desweiteren lassen diese Daten darauf schließen, dass innerhalb des H_{C}-Fragments zwei autonome Untereinheiten (Subdomänen) von jeweils 25 kDa existieren. Der erste Beweis für die Existenz der beiden funktionellen Subdomänen wurde mit der amino-terminalen (H_{CN}) und der carboxyl-terminalen Hälfte (H_{CC}) des H_{C}-Fragments des TeNT erbracht, die rekombinant exprimiert wurden und erkennen ließen, dass zwar die H_{CC}-, nicht aber die H_{CN}-Domäne an Neurone bindet (siehe Herreros et al. (2000), Biochem. J. 347, 199-204). Zu einem späteren Zeitpunkt wurde eine einzige Gangliosid-Bindungsstelle innerhalb der H_{CC}-Domänen von BoNT/A und B lokalisiert und charakterisiert (siehe Rummel et al. (2004), Mol. Microbiol. 51, 631-643). Der Ort für die Bindung des als Proteinrezeptor für BoNT/B und G identifizierten Synaptotagmins I und II konnte gleichfalls auf den Bereich der H_{CC}-Domänen von BoNT/B und G beschränkt werden (siehe Rummel et al. (2004), J. Biol. Chem. 279, 30865-70). Das Dokument offenbart jedoch nicht die für die Bindungstasche von BoNT/B und G relevanten Aminosäuren.

Unter physiologischen Bedingungen bindet die HC mit dem H_{C}-Fragment an neuronale Ganglioside, wird durch rezeptorvermittelte Endozytose ins Zellinnere aufgenommen und erreicht über das endosomale Kompartiment den natürlichen Vesikelkreislauf. Im sauren Milieu der frühen Endosomen dringt das H_{N}-Fragment in die Vesikelmembran ein und bildet eine Pore. Jede Substanz (X), die mit der HC über eine Disulfid-Brücke verbunden ist, wird durch intrazelluläre Redoxsysteme, die Zugang zur Disulfid-Brücke bekommen und sie reduzieren, von der HC getrennt werden. Letztendlich wird X im Zytosol erscheinen.

Im Falle der clostridiellen Neurotoxine ist die HC der Träger einer LC, die im finalen Schritt ihr spezifisches Substrat im Zytosol spaltet. Der Zyklus der Komplexbildung und -dissoziation der Fusionsproteine wird unterbrochen und somit die Ausschüttung von Acetylcholin gehemmt. Als Folge hiervon werden gestreifte Muskeln gelähmt, und Schweißdrüsen stellen ihre Sekretion ein. Die Wirkdauer der einzelnen BoNT Serotypen ist verschieden und hängt von der Präsenz intakter LC im Zytosol ab. Da alle Neurone Rezeptoren für clostridielle Neurotoxine besitzen, ist es nicht nur die Ausschüttung von Acetylcholin, die betroffen sein kann, sondern potentiell auch die Ausschüttung der Substanz P, von Noradrenalin, GABA, Glycin, Endorphin und anderer Transmitter und Hormone.

Dass bevorzugt die cholinerge Transmission blockiert wird, kann damit erklärt werden, dass die HC in der Peripherie in das Neuron eindringt. Zentrale Synapsen werden durch die Blut-Hirn-Schranke geschützt, die von Proteinen nicht überwunden werden kann.

In einer Ligand-Rezeptor-Studie wurden bestimmte Aminosäurereste innerhalb der H_{CC}-Domäne von BoNT/B und G ausgetauscht, um die Bindungstasche des Proteinrezeptors zu identifizieren und zu charakterisieren, um so die Affinität an den Proteinrezeptor zu verändern. Die Affinität der mutierten H_{C}-Fragmente von BoNT/B und G wurde in Gluthathion-S-Transferase-(GST)-Pulldown Experimenten mit Synaptotagmin bestimmt. Sodann wurde die HC mit denselben Mutationen an LC-B bzw. LC-G gekoppelt. Die Wirkstärke dieser Konstrukte wurde mit Hilfe des isolierten Nerv-Muskel-Präparats der Maus (Hemi-Diaphragma-Assay = HDA) analysiert. In diesem Präparat befindet sich der *Nervus phrenicus,* welcher aus cholinergen Motoneuronen besteht und das wichtigste physiologische Ziel der clostridiellen Neurotoxine darstellt. Anschließend wurden in der H_{CC}-Domäne von BoNT/A in einer Vertiefung, die sich an analoger Stelle zu den Proteinrezeptorbindungstaschen in BoNT/B und G befindet, einzelne Aminosäuren ausgetauscht. Die Voll-Längen-BoNT/A Einzelmutanten wurden anschließend ebenfalls im HDA auf veränderte Potenz analysiert, welches Hinweise auf veränderte Ligand-Proteinrezeptor-Interaktionen gibt.

Der BoNT/A-Komplex, auch Progenitortoxin A genannt, wurde in der jüngeren Vergangenheit zur Behandlung motorischer Dystonien eingesetzt, sowie zur Dämpfung exzessiver sympathischer Aktivität (siehe Benecke et al. (1995), Akt. Neurol. 22, 209ff) und zur Linderung von Schmerz und Migräne (siehe Sycha et al. (2004), J. Neurol. 251, 19-30). Dieser Komplex besteht aus dem Neurotoxin, verschiedenen Hämagglutininen und einem nicht toxischen, nicht hämagglutinierenden Protein. Unter physiologischem pH dissoziiert der Komplex in wenigen Minuten. Das hieraus hervorgehende Neurotoxin ist der einzige Bestandteil des Komplexes, der therapeutisch relevant ist und eine Linderung der Symptome verursacht. Da die zugrunde liegende neurologische Erkrankung nicht geheilt wird, muss der Komplex in Intervallen von drei bis vier Monaten erneut injiziert werden. In Abhängigkeit von der Menge des injizierten Fremdproteins bilden einige Patienten spezifische BoNT/A-Antikörper. Diese Patienten werden gegen das Neurotoxin resistent. Wenn erst einmal antigensensitive Zellen das Neurotoxin erkannt haben und Antikörper gebildet wurden, werden die diesbezüglichen Gedächtniszellen über Jahre erhalten bleiben. Darum kommt es darauf an, die Patienten mit Präparaten höchster Aktivität in möglichst geringer Dosierung zu behandeln. Die Präparate sollten ferner keine weiteren Proteine bakterieller Herkunft enthalten, da diese als Immunadjuvantien wirken können. Solche Stoffe locken Makrophagen an, die sowohl die Immunadjuvantien als auch die Neurotoxine erkennen und den Lymphozyten präsentieren, welche daraufhin mit der Bildung von Immunglobulinen antworten. Folglich sollten nur Produkte von höchster Reinheit, die keine Fremdproteine enthalten, für die Therapie Verwendung finden. Die Resistenz der Patienten gegenüber dem Neurotoxin beruht auf molekularer Ebene gesehen vorwiegend auf der Anwesenheit neutralisierender Antikörper.

Mit der vorliegenden Erfindung wird nun ein Transportprotein (Trapo) vorgestellt, das die oben geschilderten Probleme der bisher bekannten Methoden überwinden kann.

Diese Aufgabe wurde mit einem neuen Transportprotein gelöst, erhältlich durch Modifizierung der schweren Kette des von *Clostridium botulinum* gebildeten Neurotoxins, wobei
(i) das Protein an Nervenzellen mit höherer oder niedrigerer Affinität bindet als das native Neurotoxin;
(ii) das Protein im Vergleich zu dem nativen Neurotoxin eine erhöhte oder verringerte Neurotoxizität aufweist; vorzugsweise wird die Neurotoxizität im Hemidiaphragma-Assay bestimmt; und/oder
(iii) das Protein im Vergleich zu dem nativen Neurotoxin eine geringere Affinität gegenüber neutralisierenden Antikörpern aufweist.

Gemäß einer bevorzugten Ausführungsform der vorliegenden Erfindung wird ein Transportprotein bereitgestellt, das mit höherer oder niedrigerer Affinität an Nervenzellen bindet als das von C. *botulinum* gebildete native Neurotoxin.

Gemäß einem weiteren bevorzugten Ausführungsform der vorliegenden Erfindung wird ein Transportprotein bereitgestellt, welches durch Modifizierung der HC des von C. *botulinum* gebildeten Neurotoxins erhalten wird, wobei das Protein mit höherer oder niedrigerer Affinität als das native Neurotoxin spezifisch an Nervenzellen bindet. Vorzugsweise wird das Transportprotein von diesen Zellen durch Endozytose aufgenommen.

Gemäß einer weiteren bevorzugten Ausführungsform wird auch ein Transportprotein bereitgestellt, welches durch Modifizierung der HC des von *C. botulinum* gebildeten Neurotoxins erhalten wird, wobei das Protein durch Austausche oberflächenexponierter Aminosäuren insbesondere an den Gangliosid- und Proteinrezeptorbindungstaschen der, Bindung neutralisierender Antikörper nicht mehr zugänglich ist.

Nachfolgend werden Begriffe definiert, wie sie in Zusammenhang mit der vorliegenden Anmeldung zu verstehen sind.

"Bindung an Nervenzellen mit höherer oder niedrigerer Affinität als natives Neurotoxin". Das native Neurotoxin ist hierbei das native Neurotoxin von *C. botulinum.* Vorzugsweise ist das native Neurotoxin hierbei das Botulinus Neurotoxin A und/oder Botulinus Neurotoxin B und/oder Botulinus Neurotoxin G aus *C. botulinum.* Das rekombinant hergestellte Botulinus Neurotoxin aus *E. coli,* welches u. a. die zum nativen Botulinus Neurotoxin identische Aminosäuresequenz beinhaltet, verhält sich pharmakologisch identisch wie das native Botulinus Neurotoxin und wird rekombinantes Botulinus Neurotoxin Wildtyp genannt. Die hierbei erwähnten Nervenzellen sind cholinerge Motoneurone. Vorzugsweise bindet das Transportprotein spezifisch an die Plasmamembran assoziierte Moleküle, Transmembranproteine, synaptische Vesikelproteine, ein Protein der Synaptotagmin-Familie oder der synaptischen Vesikel Glykoproteine 2 (SV2), vorzugsweise Synaptotagmin I und/oder Synaptotagmin II und/oder SV2A, SV2B oder SV2C, besonders bevorzugt humanes Synaptotagmin I und/oder humanes Synaptotagmin II und/oder humanes SV2A, SV2B oder SV2C. Die Bindung wird vorzugsweise *in vitro* bestimmt. Besonders bevorzugt erfolgt die Bestimmung durch Verwendung eines GST-Pull-down-Assays, der im Detail in den Beispielen ausgeführt ist.

"das Protein weist im Vergleich zu dem nativen Neurotoxin eine erhöhte oder verringerte Neurotoxizität auf" Das native Neurotoxin ist hierbei das native Neurotoxin von *C. botulinum.* Vorzugsweise ist das native Neurotoxin hierbei das Botulinus Neurotoxin A und/oder Botulinus Neurotoxin B und/oder Botulinus Neurotoxin G aus *C*. *botulinum.* Das rekombinant hergestellte Botulinus Neurotoxin aus *E. coli,* welches u. a. die zum nativen Botulinus Neurotoxin identische Aminosäuresequenz beinhaltet, verhält sich pharmakologisch identisch wie das native Botulinus Neurotoxin und wird rekombinantes Botulinus Neurotoxin Wildtyp genannt. Die hierbei erwähnten Nervenzellen sind cholinerge Motoneurone.

Die Neurotoxizität wird vorzugsweise mit Hilfe des im Stand der Technik bekannten Hemi-Diaphragma-Assays (HDA) bestimmt. Die Neurotoxizität der Muteine kann vorzugsweise bestimmt werden wie beschrieben von Habermann et al., Naunyn Schmiedeberg's Arch. Pharmacol. 311 (1980), 33-40.

"Neutralisierende Antikörper" Gegen Botulinus Neurotoxin gerichtete neutralisierende Antikörper sind bekannt (Göschel H, Wohlfarth K, Frevert J, Dengler R, Bigalke H. Botulinum A toxin therapy: neutralizing and nonneutralizing antibodies--therapeutic consequences, Exp. Neurol. 1997 Sep;147(1):96-102). Es wurde gefunden, dass Neurotoxin neutralisierende Antikörper insbesondere mit den aktiven Zentren wie z.B. den Gangliosid- und Proteinrezeptorbindungstaschen innerhalb der H_{CC}-Domäne des Neurotoxins interagieren. Werden im Neurotoxin die die Bindungstaschen umgebenden Oberflächen durch Aminosäureaustausche verändert ohne deren Funktionalität negativ zu beeinträchtigen, verlieren die neutralisierenden Antikörper ihre Bindungsplätze und das mutierte Neurotoxin wird nicht mehr neutralisiert.

Der Ausdruck "Modifizierung der schweren Kette des von C. *botulinum* gebildeten Neurotoxins". Die Aminosäure und/oder Nukleinsäuresequenz der schweren Kette (HC) des von *C*. *botulinum* gebildeten Neurotoxins sind allgemein aus öffentlich zugänglichen Datenbanken erhältlich, für jeden der bekannten Serotypen A bis G (siehe auch Tabelle 1). Modifizierung umfasst hierbei, dass wenigstens eine Aminosäure deletiert, addiert, in die Aminosäuresequenz insertiert ist, oder wenigstens eine Aminosäure des nativen Neurotoxins durch eine andere natürliche oder nicht natürlich vorkommende Aminosäure substituiert ist und/oder eine Aminosäure in der gegebenen Aminosäuresequenz posttranslational modifiziert ist. Posttranslationale Modifikationen umfassen hierbei Glykosylierungen, Acetylierungen, Acylierungen, Desaminierungen, Phosphorylierungen, Isoprenylierungen, Glykosylphosphatidylinositolierungen, und weitere dem Fachmann bekannte Modifikationen.

Die HC des von C. *botulinum* gebildeten Neurotoxins umfasst drei Subdomänen, nämlich die amino-terminale 50 kDa große Translokationsdomäne H_{N}, die sich anschließende 25 kDa H_{CN}-Domäne und die carboxyl-terminal gelegene 25 kDa H_{CC}-Domäne. Zusammengefasst werden die H_{CN}- und H_{CC}-Domäne als H_{C}-Fragment bezeichnet. Die entsprechenden Aminosäureabschnitte der jeweiligen Subdomänen sind für die einzelnen Serotypen und ihren Varianten aus Tabelle 1 ersichtlich.

### "Gangliosidrezeptor"

Die HC der Botulinus Neurotoxine besitzen eine hohe Affinität zu peripheren Nervenzellen, die überwiegend durch die Interaktion mit komplexen Polysialogangliosiden - dies sind Glycolipide, die aus mehr als einer Sialinsäure bestehen - vermittelt wird (Halpern et al. (1995), Curr. Top. Microbiol. Immunol. 195, 221-41; WO 2006/02707). Folglich erreichen die an sie gebundenen LC nur diesen Zelltyp und werden nur in diesen Zellen wirksam. BoNT/A und B binden lediglich ein Molekül Gangliosid GT1b.

Im Falle von BoNT/B und BoNT/G sind die Proteinrezeptoren Synaptotagmin I und Synaptotagmin II. Im Falle von BoNT/A sind die Proteinrezeptoren die synaptischen Vesikel Glykoproteine 2 (SV2), vorzugsweise SV2A, SV2B und SV2C.

Zurzeit sind 13 Isoformen aus der Familie der Synaptotagmine bekannt. Alle zeichnen sich durch zwei carboxyl-terminale Ca²⁺ bindende C2-Domänen, eine mittlere Transmembrandomäne (TMD), welche das Synaptotagmin in der synaptischen Vesikelmembran verankert, und einen unterschiedlich langen Amino-Terminus aus. Nach Ca²⁺ Einstrom wird die Fusion des synaptischen Vesikels mit der Plasmamembran initiiert, woraufhin der intraluminale Amino-Terminus des Synaptotagmins extrazellulär präsentiert wird und als Rezeptoranker für BoNT/B und G zur Verfügung steht. Analog dazu steht die vierte luminale Domäne der SV2 Isoformen nach Exozytose für die Interaktion mit BoNT/A extrazellulär zur Verfügung.

Durch zielgerichtete Mutagenese wurden einzelne Aminosäuren der Bindungstasche derart in ihrem Charakter verändert, dass eine Bindung an einen Proteinrezeptor erschwert oder verhindert ist. Hierzu wurden die H_{C}-Fragmente von BoNT/B und BoNT/G rekombinant als Wildtyp oder mit einzelnen Aminosäureaustauschen (Mutationen/Substitutionen) in der postulierten Bindungstasche in *E. coli* exprimiert und isoliert. Für einen GST-Pull-down-Assay zur Untersuchung der *in vitro* Interaktion zwischen BoNT/B und BoNT/G sowie dem Synaptotagmin I und dem Synaptotagmin II wurde das jeweilige GST-Synaptotagmin-Fusionsprotein mit unterschiedlichen Mengen des jeweiligen H_{C}-Fragments von BoNT/B bzw. BoNT/G inkubiert und eine Phasentrennung durchgeführt. Freies H_{C}-Fragment verblieb im separierten Überstand, während gebundenes BoNT H_{C}-Fragment in der Festphase zusammen mit GST-Synaptotagmin-Fusionsprotein nachweisbar war. Der Ersatz der jeweiligen H_{C}-Fragmente durch die volle Länge BoNT/B und G im GST-Pull-down Assay ergab dieselben Ergebnisse.

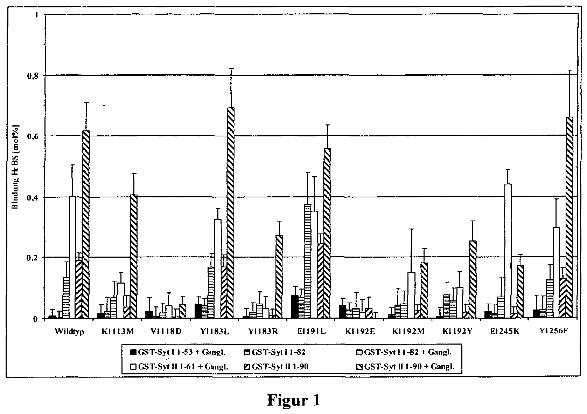

Dabei wurde herausgefunden, dass der BoNT/B Wildtyp nur in Anwesenheit von komplexen Gangliosiden und Synaptotagmin I mit Transmembrandomäne bindet, während Synaptotagmin II sowohl mit oder ohne Transmembrandomäne als auch in An- oder Abwesenheit von komplexen Gangliosiden gebunden wird. Durch zielgerichtete Substitution von Aminosäuren innerhalb der Proteinrezeptorbindungsstelle von BoNT/B konnte die Interaktion mit beiden Synaptotagmin-Molekülen deutlich gesteigert bzw. abgeschwächt werden (Figur 1).

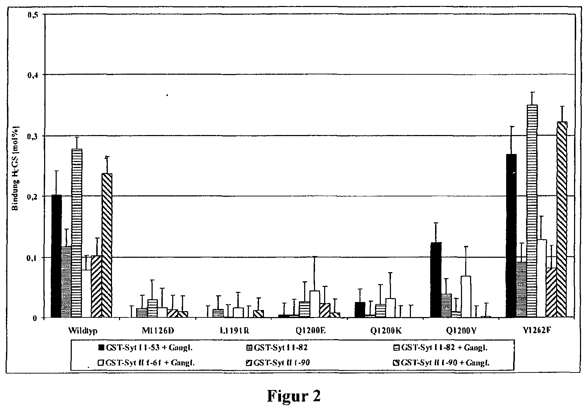

Weiterhin wurde für den BoNT/G Wildtyp gezeigt, dass sowohl in Anwesenheit als auch in Abwesenheit von komplexen Gangliosiden Bindung an Synaptotagmin I und Synaptotagmin II jeweils mit oder ohne Transmembrandomäne stattfindet. Durch zielgerichtete Substitution von zu BoNT/B homologen Aminosäuren innerhalb der Proteinrezeptorbindungsstelle von BoNT/G konnte die Interaktion mit beiden Synaptotagmin-Molekülen deutlich gesteigert bzw. abgeschwächt werden (Figur 2).

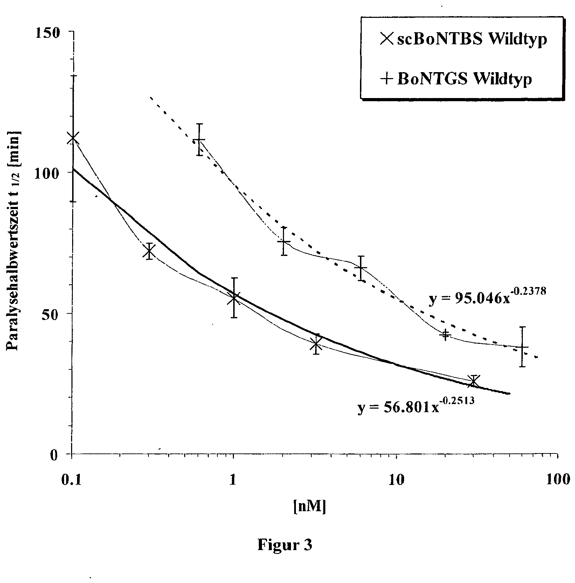

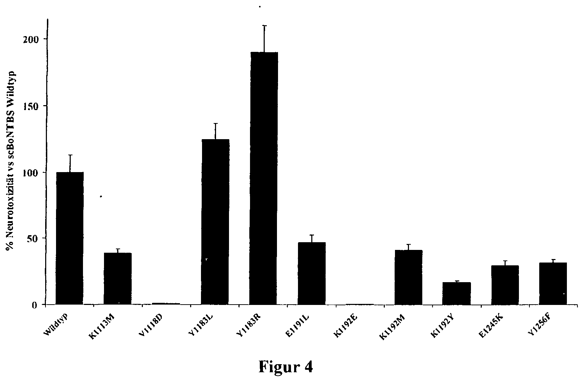

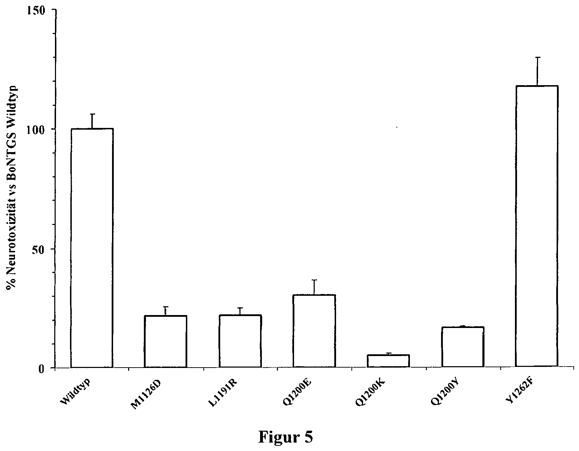

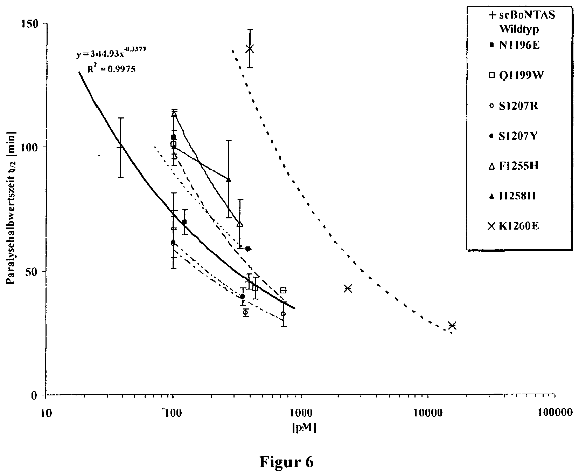

Die Potenz der Voll-Längen-Form von BoNT/A, B und G Wildtypen wurde im HDA mittels einer Dosis-Wirkungs-Kurve bestimmt (Figur 3 und 6). Anschließend wurde die Potenz der verschiedenen Voll-Längen-Formen von BoNT/A, B und G Einzelmutanten im HDA ermittelt (Figur 6) und mittels einer angelegten Potenzfunktion ins Verhältnis zur Potenz der BoNT/B und G Wildtypen gesetzt (Figur 4 und 5). Beispielhaft führt der Austausch der Aminosäuren Valin 1118 gegen Aspartat oder Lysin 1192 gegen Glutamat in BoNT/B zu einer drastischen Reduktion der Potenz auf < 2%. Im Gegensatz dazu erzeugt die Mutation des Tyrosin 1183 in Leucin bzw. Arginin eine deutliche Verstärkung der Potenz von BoNT/B (Figur 4). Modifikation von Tyrosin 1256 zu Phenylalanin in BoNT/G resultiert ebenfalls in einer Potenzzunahme während die Mutation Glutamin 1200 in Glutamat, Lysin oder Tyrosin eine starke Abnahme der Potenz von BoNT/G hervorruft (Figur 5). Im Fall von BoNT/A erzeugt die Modifikation Serin 1207 zu Arginin oder Tyrosin eine Potenzerhöhung während die Mutation Lysin 1260 zu Glutamat eine drastische Potenzverringerung des BoNT/A bewirkt (Figur 6).

Gemäß einer bevorzugten Ausführungsform weist das Transportprotein eine wenigstens 15 % höhere Affinität oder eine wenigstens 15 % niedrigere Affinität als das native Neurotoxin auf. Vorzugsweise weist das Transportprotein eine wenigstens 50 % höhere oder niedrigere, besonders bevorzugt wenigstens 80 % höhere oder niedrigere, und insbesondere eine wenigstens 90 % höhere oder niedrigere Affinität als das native Neurotoxin auf.

Gemäß einer bevorzugten Ausführungsform erfolgt die Modifizierung der HC im Bereich des H_{C}-Fragmentes des gegebenen Neurotoxins. Sofern die Modifikation eine Substitution, Deletion, Insertion oder Addition umfasst, kann diese beispielsweise durch zielgerichtete Mutagenese durchgeführt werden, Verfahren hierzu sind dem Fachmann bekannt. Die in dem nativen Neurotoxin vorhandenen Aminosäuren werden hierbei entweder durch natürlich vorkommende oder nicht natürlich vorkommende Aminosäuren verändert. Grundsätzlich werden Aminosäuren in unterschiedliche physikochemische Gruppen eingeteilt. Zu den negativ geladenen Aminosäuren gehören Aspartat und Glutamat. Zu den positiv geladenen Aminosäuren gehören Histidin, Arginin und Lysin. Zu den polaren Aminosäuren gehören Asparagin, Glutamin, Serin, Threonin, Cystein und Tyrosin. Zu den unpolaren Aminosäuren gehören Glycin, Alanin, Valin, Leucin, Isoleucin, Methionin, Prolin, Phenylalanin und Tryptophan. Aromatische Seitengruppen finden sich bei den Aminosäuren Histidin, Phenylalanin, Tyrosin und Tryptophan. Generell ist bevorzugt, dass eine Aminosäure durch eine andere Aminosäure ausgetauscht wird, die zu einer anderen physikochemischen Gruppe gehört.

Gemäß einer bevorzugten Ausführungsform der Erfindung ist das Transportprotein ein Botulinus Neurotoxin Serotyp A bis G. Hierbei sind die Aminosäuresequenzen der nativen Neurotoxine wie folgt aus öffentlich zugänglichen Datenbanken erhältlich:

**Tabelle 1: Datenbanknummem der Aminosäuresequenzen und Aufteilung der Subdomänen der sieben Botulinus Neurotoxine.**

| BoNT | Datenbanknr. der Proteinsequenz | Anzahl der Aminosäuren | HC | | |
|---|---|---|---|---|---|
| | | | H_{N} | H_{C} | |
| | | | | H_{CN} | H_{CC} |
| BoNT/A | AAA23262 | 1296 | 449-866 | 867-1091 | 1092-1296 |
| | AAM75961 | | | | |
| | AAQ06331 | | | | |
| | BTCLAB | | | | |
| | P10845 | 1296 | 449-866 | 867-1091 | 1092-1296 |
| | CAA36289 | 1296 | 449-866 | 867-1091 | 1092-1296 |
| | CAA51824 | 1296 | 449-866 | 867-1091 | 1092-1296 |
| | 140645 | | | | |
| | Q45894 | | | | |
| BoNT/B | AAL11499 | 1291 | 442-855 | 866-1078 | 1079-1291 |
| | AAL11498 | | | | |
| | CAA73968 | 1291 | 442-855 | 866-1078 | 1079-1291 |
| | AAK971.32 | 1291 | 442-855 | 866-1078 | 1079-1291 |
| | A48940 | 1291 | 442-855 | 866-1078 | 1079-1291 |
| | AAA23211 | | | | |
| | P10844 | | | | |
| | BAC22064 | 1291 | 442-855 | 866-1078 | 1079-1291 |
| | CAA50482 | 1291 | 442-855 | 866-1078 | 1079-1291 |
| | I40631 | | | | |
| BoNT/C1 | A49777 | 1291 | 450-863 | 864-1092 | 1093⁻1291 |
| | BAA14235 | | | | |
| | BAB71749 | | | | |
| | CAA51313 | | | | |
| | S46431 | | | | |
| | P18640 | 1291 | 450-863 | 864-1092 | 1093-1291 |
| | BAA08418 | 1280 | 450-863 | 864-1083 | 1084-1280 |
| | BAA89713 | 1280 | 450-863 | 864-1083 | 1084-1280 |
| BoNT/D | CAA38175 | 1276 | 446-859 | 860-1079 | 1080-1276 |
| | P19321 | | | | |
| | S11455 | | | | |
| | AAB24244 | 1276 | 446-859 | 860-1079 | 1080-1276 |
| | BAA07477 | 1285 | 446-859 | 860-1088 | 1089-1285 |
| | S70582 | | | | |
| | BAA90661 | 1285 | 446-859 | 860-1088 | 1089-1285 |
| BoNT/E | BAB86845 | 1252 | 423-842 | 843-1066 | 1067-1252 |
| | CAA44558 | | | | |
| | S21178 | | | | |
| | CAA43999 | 1251 | 423-842 | 843-1066 | 1067-1251 |
| | Q00496 | | | | |
| | CAA43998 | 1251 | 423-842 | 843-1066 | 1067-1251 |
| | JH0256 | | | | |
| | P30995 | | | | |
| BoNT/F | 1904210A | 1274 | 440-860 | 861-1086 | 1087-1274 |
| | AAA23263 | | | | |
| | I40813 | | | | |
| | P30996 | | | | |
| | CAA73972 | 1280 | 440-861 | 862-1087 | 1088-1280 |
| | AAA23210 | 1278 | 440-861 | 862-1084 | 1085-1278 |
| | CAA57358 | | | | |
| | CAA48329 | 1268 | 432-853 | 854-1075 | 1076-1268 |
| | S33411 | | | | |
| BoNT/G | CAA52275 | 1297 | 447-860 | 861-1086 | 1087-1297 |
| | Q60393 | | | | |
| | S39791 | | | | |

Im Hinblick auf das H_{C}-Fragment dieser Botulinus Neurotoxine sind für eine Modifizierung bevorzugt die Aminosäuren in den Aminosäurepositionen von
867 bis 1296 des *C*. *botulinum* Neurotoxin Serotyp A,
866 bis 1291 des *C*. *botulinum* Neurotoxin Serotyp B,
864 bis 1291 bzw. 1280 des *C*. *botulinum* Neurotoxin Serotyp Cl,
860 bis 1276 bzw. 1285 des *C*. *botulinum* Neurotoxin Serotyp D,
843 bis 1251 bzw. 1252 des *C*. *botulinum* bzw. *C*. *butyricum* Neurotoxin Serotyp E,
861 bis 1274, 862 bis 1280 bzw. 1278 und 854 bis 1268 des *C*. *botulinum* bzw. *C. baratii* Neurotoxin Serotyp F,
861 bis 1297 des *C*. *botulinum* Neurotoxin Serotyp G.

Es ist daher bevorzugt, dass mindestens eine Aminosäure in den vorgenannten Positionen posttranslational modifiziert, und/oder addiert, und/oder deletiert, und/oder insertiert, und/oder durch eine Aminosäure substituiert ist, die entweder natürlich vorkommt oder nicht natürlichen Ursprungs ist.

Gemäß einer bevorzugten Ausführungsform ist das Neurotoxin Botulinus Neurotoxin Serotyp A. Vorzugsweise wird hierbei mindestens eine Aminosäure in den Positionen Threonin 1195, Asparagin 1196, Glutamin 1199, Lysin 1204, Isoleucin 1205, Leucin 1206, Serin 1207, Leucin 1209, Aspartat 1213, Leucin 1217, Phenylalanin 1255, Asparagin 1256, Isoleucin 1258 und/oder Lysin 1260 der Botulinus Neurotoxin Serotyp A. Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist. Besonders bevorzugt sind die Positionen Asparagin 1196, Glutamin 1199, Serin 1207, Phenylalanin 1255, Isoleucin 1258 und/oder Lysin 1260der Botulinus Neurotoxin Serotyp A Proteinsequenzen. Insbesondere sind die Positionen Serin 1207, welches durch Arginin oder Tyrosin ersetzt wird, und Lysin 1260, das gegen Glutamat ausgetauscht wird, bevorzugt.

Gemäß einer bevorzugten Ausführungsform ist das Neurotoxin Botulinus Neurotoxin Serotyp B. Vorzugsweise wird hierbei mindestens eine Aminosäure in den Positionen Lysin 1113, Aspartat 1114, Serin 1116, Prolin 1117, Valin 1118, Threonin 1182, Tyrosin 1183, Phenylalanin 1186, Lysin 1188, Glutamat 1191, Lysin 1192, Leucin 1193, Phenylalanin 1194, Phenylalanin 1204, Phenylalanin 1243, Glutamat 1245, Lysin 1254, Aspartat 1255 und Tyrosin 1256 der Botulinus Neurotoxin Serotyp B Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist. Besonders bevorzugt sind die Positionen Valin 1118, Tyrosin 1183, Glutamat 1191, Lysin 1192, Glutamat 1245 und Tyrosin 1256 der Botulinus Neurotoxin Serotyp B Proteinsequenzen. Insbesondere sind die Positionen Tyrosin 1183 und Glutamat 1191, welche durch Leucin ersetzt werden, bevorzugt.

Gemäß einer weiteren bevorzugten Ausführungsform ist das Neurotoxin Botulinus Neurotoxin Serotyp G. Vorzugsweise wird hierbei mindestens eine Aminosäure in den Positionen Phenylalanin 1121, Lysin 1123, Alanin 1124, Serin 1125, Methionin 1126, Valin 1190, Leucin 1191, Serin 1194, Glutamat 1196, Threonin 1199, Glutamin 1200, Leucin 1201, Phenylalanin 1202, Phenylalanin 1212, Phenylalanin 1248, Lysin 1250, Aspartat 1251 und Tyrosin 1262 der Botulinus Neurotoxin Serotyp G Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist. Besonders bevorzugt sind die Positionen Methionin 1126, Leucin 1191, Threonin 1199, Glutamin 1200, Lysin 1250 und Tyrosin 1262 der Botulinus Neurotoxin Serotyp G Proteinsequenzen. Insbesondere ist die Position Tyrosin 1262, welche durch Phenylalanin ersetzt wird, bevorzugt.

Das in der vorliegenden Erfindung bereitgestellte Transportprotein weist eine erhöhte oder verringerte spezifische Affinität seiner proteinbindenden Domäne insbesondere zu Molekülen aus der Familie der Synaptotagmine oder der synaptischen Vesikel Glykoproteine 2 auf.

Eine weitere Ausführungsform der vorliegenden Erfindung bezieht sich auf eine Zusammensetzung, die ein erfindungsgemäßes Transportprotein und wenigstens ein intervenierendes Molekül (X) enthält. Das intervenierende Molekül kann ein kleines organisches Molekül, ein Peptid oder ein Protein sein; vorzugsweise kovalent durch eine Peptid-Bindung, Ester-Bindung, Ether-Bindung, Sulfid-Bindung, Disulfid-Bindung oder Kohlenstoff-Kohlenstoff-Bindung an das Transportprotein gebunden.

Das intervenierende Molekül umfasst zusätzlich alle bekannten therapeutisch wirksamen Stoffe. Bevorzugt sind hierbei Zytostatika, Antibiotika, Virustatika, aber auch Immunglobuline.

In einer bevorzugten Ausführungsform ist das Protein eine Protease, die ein oder mehrere Proteine des Freisetzungsapparates von Neurotransmittern spaltet, wobei die Protease aus der Gruppe der Neurotoxine ausgewählt ist, die aus den LC der *C. botulinum* Neurotoxine, insbesondere des Serotyps A, B, Cl, D, E, F und G oder einem proteolytisch aktivem Fragment der LC eines *C. botulinum* Neurotoxins, insbesondere eines Neurotoxins des Serotyps A, B, Cl, D, E, F und G besteht, wobei das Fragment wenigstens 0,01 % der proteolytischen Aktivität der nativen Protease, vorzugsweise wenigstens 5 %, besonders bevorzugt wenigstens 50 %, insbesondere wenigstens 90 % aufweist. Vorzugsweise ist das Transportprotein und die Protease vom gleichen *C*. *botulinum* Neurotoxin Serotyp abgeleitet, insbesondere bevorzugt ist die H_{N}-Domäne des Transportproteins und die Protease vom *C*. *botulinum* Neurotoxin Serotyp A abgeleitet. Die Sequenzen der Proteasen sind allgemein aus Datenbanken zugänglich und die Datenbanknummern sind aus Tabelle 1 ersichtlich. Die proteolytische Aktivität der Proteasen wird anhand einer Substratspaltungskinetik bestimmt (siehe Binz et al. (2002), Biochemistry 41(6), 1717-23).

Gemäß einer weiteren Ausführungsform der vorliegenden Erfindung wird ein Verfahren zur Herstellung des Transportproteins bereitgestellt. In einem ersten Schritt wird hierbei eine das Transportprotein kodierende Nukleinsäure bereitgestellt. Die kodierende Nukleinsäure kann hierbei RNA, DNA oder Gemische davon darstellen. Die Nukleinsäure kann ferner im Hinblick auf ihre Nukleaseresistenz modifiziert sein wie z. B. durch Einfügung von Phosphorthioat-Bindungen. Die Nukleinsäure kann aus einer Ausgangs-Nukleinsäure hergestellt werden, wobei die Ausgangs-Nukleinsäure z. B. durch Klonierung aus genomischen oder cDNA-Banken zugänglich ist. Weiterhin kann die Nukleinsäure direkt durch Festphasensynthese hergestellt werden. Geeignete Verfahren sind dem Fachmann bekannt. Sofern von einer Ausgangs-Nukleinsäure ausgegangen wird, kann z. B. durch ortsgerichtete Mutagenese eine zielgerichtete Veränderung durchgeführt werden, die auf Aminosäure-Ebene zu wenigstens einer Addition, Insertion, Deletion und/oder Substitution führt. Die Nukleinsäure wird sodann in operativer Weise mit einem geeigneten Promotor verbunden. Geeignete Promotoren für die Expression in bekannten Expressionssystemen sind dem Fachmann bekannt. Die Wahl des Promotors hängt hierbei vom zur Expression verwendeten Expressionssystem ab. Generell sind konstitutive Promotoren bevorzugt, jedoch sind auch induzierbare Promotoren verwendbar. Das so hergestellte Konstrukt umfasst wenigstens einen Teil eines Vektors, insbesondere regulatorische Elemente, wobei der Vektor beispielsweise ausgewählt ist aus λ-Derivaten, Adenoviren, Baculoviren, Vacciniaviren, SV40-Viren und Retroviren. Der Vektor ist vorzugsweise zur Expression der Nukleinsäure in einer gegebenen Wirtszelle fähig.

Ferner stellt die Erfindung Wirtszellen bereit, die den Vektor enthalten und die zur Expression des Vektors geeignet sind. Im Stand der Technik sind zahlreiche prokaryontische und eukaryontische Expressionssysteme bekannt, wobei die Wirtszellen beispielsweise ausgewählt sind aus prokaryontischen Zellen wie *E. coli* oder *B. subtilis,* aus eukaryontischen Zellen wie *S. cerevisiae* und *P. pastoris.* Zwar können auch höhere eukaryontischen Zellen verwendet werden wie Insektenzellen oder Säugerzellen, jedoch sind Wirtszellen bevorzugt, die ebenso wie *C*. *botulinum* über keinen Glykosylierungs-Apparat verfügen.

Gemäß einer bevorzugten Ausführungsform kodiert die Nukleinsäure das H_{C}-Fragment des *C. botulinum* Neurotoxins. Diese Nukleinsäure enthält Endonuklease-Schnittstellen, die die H_{C}-Fragment kodierende Nukleinsäure flankieren, wobei die Endonukleasestellen kompatibel sind mit denen anderer H_{C}-Fragmente aus *C*. *botulinum* Neurotoxinen, um deren leichten modularen Austausch in dem das Transportprotein codierenden Gen zu gestatten, während die Ähnlichkeit der Aminosäuresequenz erhalten bleibt.

Sofern eine erfindungsgemäße Zusammensetzung bereitgestellt wird, die neben dem Transportprotein noch wenigstens ein intervenierendes Molekül enthält, und dieses intervenierende Molekül ein Peptid oder Protein entweder mit einem carboxyl-terminalen Cystein oder einer Mercapto-Gruppe funktionalisiert ist, so kann in analoger Weise wie zuvor beschrieben das Peptid bzw. das Protein rekombinant hergestellt werden, beispielsweise unter Verwendung von binären Vektoren oder durch unterschiedliche Wirtszellen. Sofern dieselbe Wirtszelle für die Expression sowohl des Transportproteins als auch des Peptids oder Proteins verwendet wird, wird vorzugsweise eine intermolekulare Disulfid-Bindung *in situ* gebildet. Zur effizienteren Produktion in derselben Wirtszelle kann die das Peptid oder Protein kodierende Nukleinsäure auch mit der des Transportproteins im gleichen Leserahmen translatiert werden, so dass ein einkettiges Polypeptid produziert wird. Hierbei bildet sich dann vorzugsweise eine intramolekulare Disulfid-Bindung *in situ.* Zur einfachen Hydrolyse der ebenfalls vorhanden peptidischen Verknüpfung zwischen Transportprotein und Peptid bzw. Protein wird am Amino-Terminus des Transportproteins eine Aminosäuresequenz, die entweder spezifisch von der Protease Thrombin oder einer spezifischen Endoprotease der Wirtszelle erkannt und gespalten wird, eingefügt.

Überraschenderweise wurde festgestellt, dass die Insert-Sequenz CXXXZKTKSLVPRGSKBXXC (SEQ ID NO:1), wobei X eine beliebige Aminosäure bedeutet und Z und B unabhängig voneinander ausgewählt ist aus Alanin, Valin, Serin, Threonin und Glycin, von einer endogenen Protease eines bakteriellen Wirtes, vorzugsweise *E. coli* effizient *in vivo* gespalten wird. Das Einfügen der Insertsequenz zwischen die Aminosäuresequenz des Transportproteins und eines weiteren Peptids oder Proteins hat daher den Vorteil, dass eine spätere Nachprozessierung wie z.B. durch Thrombin nicht erforderlich ist. Besonders bevorzugt ist der *E. coli-Stamm E. coli* K12.

Die Insertsequenz ist vorzugsweise Teil eines Loops mit 18 20, vozugsweise Aminosäuren.

Sofern dieses nicht möglich ist, kann nach separater Aufreinigung des Transportproteins und des Proteins nachfolgend durch dem Fachmann bekannte Oxidationsverfahren eine entsprechende intermolekulare Disulfid-Bindung herbeigeführt werden. Das Peptid bzw. Protein kann auch direkt durch Synthese oder Fragmentkondensation erhalten werden. Entsprechende Verfahren sind dem Fachmann bekannt.

Das Transportprotein und das Peptid bzw. Protein werden nachfolgend aufgereinigt. Hierbei kommen dem Fachmann bekannte Verfahren zum Einsatz, wie z. B. Chromatographie-Verfahren oder Elektrophorese.

Eine weitere Ausführungsform der vorliegenden Erfindung bezieht sich auf die pharmazeutische Zusammensetzung, die das Transportprotein oder eine Zusammensetzung und optional einen pharmazeutisch akzeptablen Träger, ein Verdünnungsmittel und/oder Additiv enthält.

Die pharmazeutische Zusammensetzung ist zur oralen, intravenösen, subkutanen, intramuskulären und topischen Verabreichung geeignet. Die intramuskuläre Verabreichung ist hierbei bevorzugt. Eine Dosiseinheit der pharmazeutischen Zusammensetzung enthält ungefähr 0,1 pg bis 1 mg Transportprotein und/oder die erfindungsgemäße Zusammensetzung.

Die pharmazeutische Zusammensetzung ist zur Behandlung von Störungen der Neurotransmitterfreisetzung und von Erkrankungen wie z. B. Hemifacialspasmus, spasmodischer Torticollis, Blepharospasmus, Spastizitäten, Dystonien, Migräne, Schmerzen, Erkrankungen,der Hals- und Lendenwirbelsäule, Strabismus, Hypersalivation, Wundheilung, Schnarchen und Depressionen geeignet.

Eine weitere Ausführungsform der vorliegenden Erfindung schließt eine kosmetische Zusammensetzung ein, die ein Transportprotein und einen kosmetisch akzeptablen Träger, Verdünnungsmittel und/oder Additiv enthält. Die kosmetische Zusammensetzung ist geeignet für die Behandlung von Hyperhydrose und Gesichtsfalten.
- Figur 1:: Untersuchung der *in vitro* Bindung der Wildtyp und mutierten BoNT/B H_{C}-Fragmente an verkürzten GST-Syt I und GST-Syt II Fusionsproteinen in Gegenwart oder Abwesenheit von komplexen Gangliosiden mittels GST-Pull-down Assay.
- Figur 2:: Untersuchung der *in vitro* Bindung der Wildtyp und mutierten BoNT/G H_{C}-Fragmente an verkürzten GST-Syt I und GST-Syt II Fusionsproteinen in Gegenwart oder Abwesenheit von komplexen Gangliosiden mittels GST-Pull-down Assay.
- Figur 3:: Dosis-Wirkungs-Kurve der BoNT/B und G Wildtypen im HDA. Die angelegten Potenzfunktionen ermöglichen einen relativen Vergleich der Paralysezeiten der Einzelmutanten mit denen der dazugehörigen Wildtypen.
- Figur 4:: Ab- und Zunahme der Neurotoxizität der BoNT/B Einzelmutanten im Vergleich zum Wildtyp im HDA.
- Figur 5:: Ab- und Zunahme der Neurotoxizität der BoNT/G Einzelmutanten im Vergleich zum Wildtyp im HDA.
- Figur 6:: Dosis-Wirkungs-Kurven des BoNT/A Wildtyps und der BoNT/A Einzelmutanten im HDA.

Im Detail beinhaltet die vorliegende Erfindung ein Transportprotein (Trapo), das durch Modifizierung der HC des von *C*. *botulinum* produzierten Neurotoxins entsteht, vorzugsweise spezifisch an Neurone bindet, und vorzugsweise durch rezeptorvermittelte Endozytose intrazellulär aufgenommen wird und aus dem sauren endosomalen Kompartiment in das Zytosol von Neuronen transloziert wird. Dieses Protein wird als Transporter benutzt, um daran gebundene Proteasen und andere Substanzen, die physiologisch die Plasmamembran nicht durchdringen und in das Zytosol von Nervenzellen gelangen können, in die Zellen einzuschleusen. Die Substrate der Proteasen sind intrazellulär lokalisierte Proteine und Peptide, die an der Transmitterfreisetzung beteiligt sind. Nach Spaltung der Substrate sind die spezifischen Funktionen der Neurone blockiert, wobei die Zellen selbst nicht geschädigt sind. Eine dieser Funktionen ist die Exozytose, die die Neurotransmitterfreisetzung bewerkstelligt. Ist die Freisetzung von Transmittern gehemmt, ist die Übermittlung von Signalen von Zelle zu Zelle blockiert. Beispielsweise werden gestreifte Muskeln gelähmt, wenn die Freisetzung von Acetylcholin an der neuromuskulären Kontaktstelle gehemmt ist. Diese Wirkung kann therapeutisch genutzt werden, wenn das Transportprotein an Nervenendigungen spastischer oder dystoner Muskeln appliziert wird. Andere aktive Substanzen sind beispielsweise Wirkstoffe mit antiviraler Wirkung. Mit dem Transportprotein konjugiert, sind sie für die Behandlung viraler Infektionen des Nervensystems von Nutzen. Die vorliegende Erfindung bezieht sich auch auf die Verwendung eines Transportproteins zur Hemmung der Freisetzung von Neurotransmittern.

Transportproteine mit niedrigerer Affinität binden an die Nervenzellen, werden jedoch von diesen nicht aufgenommen. Diese Transportproteine eignen sich daher als spezifische Transporter zur Nervenzelloberfläche.

Wenn Patienten mit den Progenitortoxinen A und B aus *C. botulinum* behandelt werden, verursacht die Injektion dieser nicht humanen Proteine trotz der niedrigen Dosis die Bildung von Antikörpern, so dass die Therapie wirkungslos bleibt und daher abgebrochen werden muss, um letztendlich einen anaphylaktischen Schock zu vermeiden. Indem man eine Substanz desselben Wirkmechanismus mit einer höheren Transporteffizienz der enzymatischen Aktivität appliziert, kann die Dosis drastisch gesenkt werden, und es wird nicht zur Bildung von Antikörpern kommen. Diese Eigenschaften kommen dem hier beschriebenen Transportprotein zu.

Auch wenn Beispiele für die Applikation angegeben werden, so werden im Allgemeinen der geeignete Applikationsmodus und die Dosierung von dem behandelnden Arzt individuell bestimmt. Solche Entscheidungen werden routinemäßig von jedem im Fachgebiet bewanderten Arzt getroffen. So kann z. B. der Applikationsmodus und die Dosierung des Neurotoxins gemäß der hier dargelegten Erfindung auf der Basis von Kriterien wie der Löslichkeit des gewählten Neurotoxins oder der Intensität der zu behandelnden Schmerzen gewählt werden.

Gegenwärtig beträgt das Behandlungsintervall für die nativen Progenitortoxine A und B aus *C*. *botulinum* durchschnittlich drei bis vier Monate. Die Verlängerung dieses Intervalls würde das Risiko der Antikörperbildung vermindern und eine längere Dauer der Behandlung mit BoNT ermöglichen. Die Zunahme von LC im Zytosol würde deren Abbau zeitlich strecken und damit auch die Wirkdauer verlängern. Das hier beschriebene Transportprotein besitzt eine höhere Affinität und Aufnahmerate als die native HC.

Das folgende Beispiel dient lediglich dem Zwecke der Verdeutlichung und sollte nicht als begrenzend angesehen werden.

### Material und Methoden

### Plasmidkonstruktion und Herstellung rekombinanter Proteine

Plasmide für die *E. coli* Expression von rekombinanten H_{C}-Fragmenten von BoNT/B und BoNT/G sowie der Voll-Längen-Form von BoNT/A, B und G mit carboxyl-terminalen StrepTag zur Affinitätsaufreinigung wurden durch PCR-Verfahren mit geeigneten Primern, BoNT/A (AAA23262) BoNT/B (AAA23211) und BoNT/G (CAA52275) kodierender chromosomaler DNA und dem Expressionsvektor pQe3 (Qiagen AG) als Ausgangsvektor erzeugt. Verkürzte Varianten von Ratten-Synaptotagmin I (Syt I) (Aminosäuren 1-53; Aminosäuren 1-82) und Ratten-Synaptotagmin II (Syt II) (Aminosäuren 1-61; Aminosäuren 1-90) wurden in den GST kodierenden Vektor pGEX-2T (Amersham Biosciences AB) einkloniert. Die Nukleinsäuresequenzen sämtlicher Plasmide wurden durch DNA-Sequenzierung bestätigt. Die rekombinanten H_{C}-Fragmente und die der Voll-Längen-Form von BoNT wurden im E. *coli*-Stamm M15 [pRep4] (Qiagen) während einer zehnstündigen Induktion bei Raumtemperatur hergestellt und an einer StrepTactin-Matrix (IBA GmbH) gemäß den Herstellerempfehlungen aufgereinigt. Die aus *E*. *coli* BL21 erhaltenen GST-Fusionsproteine wurden mit Hilfe von auf Sepharosekügelchen immobilisierten Glutathion isoliert. Fraktionen, die die gewünschnten Proteine enthielten, wurden vereinigt und gegen Tris-NaCl-Triton-Puffer (20 mM Tris-HCl, 150 mM NaCl, 0,5 % Triton X-100, pH 7,2) dialysiert.

### GST-Pull-down Assay

GST-Fusionsproteine (jeweils 0,12 nmol), die auf 10 µl GT-SepharoseKügelchen immobilisiert waren, wurden mit H_{C}-Fragmenten (0,1 nmol) in Abwesenheit oder Gegenwart eines Rinderhirn-Gangliosid-Gemisches (18% GM1, 55 % GDla, 10 % GT1b, 2 % sonstige Gangloside; Calbiochem; 20 µg jeweils) in einem Gesamtvolumen von 180 µl Tris-NaCl-Triton-Puffer für 2 h bei 4 °C inkubiert. Die Kügelchen wurden durch Zentrifugation gesammelt, der Überstand entfernt und die separierten Kügelchen jeweils dreimal mit 400 µl des gleichen Puffers gewaschen. Die gewaschenen Pellet-Fraktionen wurden in SDS-Probenpuffer aufgekocht und zusammen mit den Überstandfraktionen durch SDS-PAGE und Coomassie Blaufärbung untersucht.

Der BoNT/B Wildtyp bindet nur in Anwesenheit von komplexen Gangliosiden und Synaptotagmin I mit Transmembrandomäne, während Synaptotagmin II sowohl mit oder ohne Transmembrandomäne als auch in An- oder Abwesenheit von komplexen Gangliosiden gebunden wird. Durch zielgerichtete Substitution von Aminosäuren innerhalb der Proteinrezeptorbindungsstelle von BoNT/B konnte die Interaktion mit beiden Synaptotagmin-Molekülen deutlich gesteigert (E1191L; Y1183L) bzw. abgeschwächt (V1118D; K1192E) werden (Figur 1).

Für den BoNT/G Wildtyp wurde gezeigt, dass sowohl in Anwesenheit als auch in Abwesenheit von komplexen Gangliosiden Bindung an Synaptotagmin I und Synaptotagmin II jeweils mit oder ohne Transmembrandomäne stattfindet. Durch zielgerichtete Substitution von zu BoNT/B homologen Aminosäuren innerhalb der Proteinrezeptorbindungsstelle von BoNT/G konnte die Interaktion mit beiden Synaptotagmin-Molekülen deutlich gesteigert (Y1262F) bzw. abgeschwächt (Q1200E) werden (Figur 2).

Durch Nachweis der Bindung der rekombinanten H_{C}-Fragmente von BoNT/B und G an isolierte, immobilisierte Gang1 ioside konnte eine Schädigung der Funktion der benachbarten Gangliosid-Bindungstasche durch die in der Syt-Bindungstasche eingeführten Mutationen ausgeschlossen und ausreichende Rückschlüsse auf eine intakte Tertiärstruktur des H_{C}-Fragments geschlossen werden. Diese Ergebnisse werden unterstützt durch CD-spektroskopische Untersuchungen sowie thermischer Denaturierungsexperimente, welche ebenfalls intakte Tertiärstrukturen der mutierten H_{C}-Fragmente von BoNT/B und G anzeigen.

### Maus Hemidiaphragma Assay (HDA)

Die Neurotoxizität der BoNT/A, B und G-Muteine wurde bestimmt wie beschrieben von Habermann et al., Naunyn Schmiedeberg's Arch. Pharmacol. 311 (1980), 33-40.

Die Potenz der Voll-Längen-Form von BoNT/A, B und G Wildtypen wurde im HDA mittels einer Dosis-Wirkungs-Kurve bestimmt (Figur 3 und 6). Anschließend wurde die Potenz der verschiedenen Voll-Längen-Formen von BoNT/A, B und G Einzelmutanten im HDA ermittelt (Figur 6) und mittels einer angelegten Potenzfunktion ins Verhältnis zur Potenz der BoNT/B und G Wildtypen gesetzt (Figur 4 und 5). So führt der Austausch der Aminosäuren Valin 1118 gegen Aspartat oder Lysin 1192 gegen Glutamat in BoNT/B zu einer drastischen Reduktion der Potenz auf < 2%. Im Gegensatz dazu erzeugt die Mutation des Tyrosin 1183 in Leucin bzw. Arginin eine deutliche Verstärkung der Potenz von BoNT/B (Figur 4). Modifikation von Tyrosin 1256 zu Phenylalanin in BoNT/G resultiert ebenfalls in einer Potenzzunahme während die Mutation Glutamin 1200 in Glutamat, Lysin oder Tyrosin eine starke Abnahme der Potenz von BoNT/G hervorruft (Figur 5). Im Fall von BoNT/A erzeugt die Modifikation Serin 1207 zu Arginin oder Tyrosin eine Potenzerhöhung während die Mutation Lysin 1260 zu Glutamat eine drastische Potenzverringerung des BoNT/A bewirkt (Figur 6).

Weitere bevorzugte Ausfuehrungsformen sind nachfolgend beschrieben.
1. Ein Transportprotein, erhältlich durch Modifizierung der schweren Kette des von *Clostridium botulinum* gebildeten Neurotoxins, wobei
   (i) das Protein an Nervenzellen mit höherer oder niedrigerer Affinität bindet als das native Neurotoxin;
   (ii) das Protein im Vergleich zu dem nativen Neurotoxin eine erhöhte oder verringerte Neurotoxizität aufweist; vorzugsweise wird die Neurotoxizität im Hemidiaphragma-Assay bestimmt; und/oder
   (iii) das Protein im Vergleich zu dem nativen Neurotoxin eine geringere Affinität gegenüber neutralisierenden Antikörpern aufweist.
2. Das Transportprotein nach 1, wobei der neutralisierende Antikörper die Bindung des nativen Neurotoxins an den Proteinrezeptor oder Gangliosidrezeptor verhindert und/oder die Aufnahme des Neurotoxins in die Nervenzelle verhindert.
3. Das Transportprotein nach 1 oder 2, wobei das Transportprotein durch Endozytose von den Zellen aufgenommen wird.
4. Das Transportprotein nach einem der 1 bis 3, wobei das Protein spezifisch an die Plasmamembran assoziierte Moleküle, Transmembranproteine, synaptische Vesikelproteine, Proteine der Synaptotagmin-Familie oder der synapatischen Vesikel Glykoproteine 2 (SV2) und/oder Synaptotagmin I und/oder Synaptotagmin II (cholinerger Motoneurone) und/oder SV2A, SV2B oder SV2C bindet, bevorzugt humanes Synaptotagmin I und/oder humanes Synaptotagmin II und/oder humanes SV2A, SV2B oder SV2C.
5. Das Transportprotein nach einem der 1 bis 4, wobei das Protein eine wenigstens 15 % höhere Affinität oder eine wenigstens 15 % niedrigere Affinität als das native Neurotoxin aufweist, vorzugsweise wenigstens 50 %, besonders bevorzugt wenigstens 80 %, insbesondere bevorzugt wenigstens 90 %.
6. Das Transportprotein nach einem der 1 bis 5, wobei das H_{C}-Fragment des Transportproteins wenigstens eine Substitution und/oder Deletion und/oder Insertion und/oder Addition und/oder posttranslationale Modifikationen von Aminosäuren, die entweder natürlich vorkommen oder nicht natürlichen Ursprungs sind und die die Affinität gegenüber dem nativen Neurotoxin erhöhen oder erniedrigen, aufweist.
7. Das Transportprotein nach einem der 1 bis 6, wobei das Neurotoxin Botulinus Neurotoxin Serotyp A bis G ist.
8. Das Transportprotein nach 7, wobei mindestens eine Aminosäure in den Aminosäurepositionen
   867 bis 1296 des *Clostridium botulinum* Neurotoxin Serotyp A,
   866 bis 1291 des *Clostridium botulinum* Neurotoxin Serotyp B,
   864 bis 1291 bzw. 1280 des *Clostridium botulinum* Neurotoxin Serotyp C1,
   860 bis 1276 bzw. 1285 des *Clostridium botulinum* Neurotoxin Serotyp D,
   843 bis 1251 bzw. 1252 des *Clostridium botulinum* bzw. *Clostridium butyricum* Neurotoxin Serotyp E,
   861 bis 1274, 862 bis 1280 bzw. 1278 und 854 bis 1268 des *Clostridium botulinum* bzw. *Clostridium baratii* Neurotoxin Serotyp F,
   861 bis 1297 des *Clostridium botulinum* Neurotoxin Serotyp G. posttranslational modifiziert, und/oder addiert, und/oder deletiert, und/oder insertiert und/oder durch Aminosäuren substituiert ist, die entweder natürlich vorkommen oder nicht natürlichen Ursprungs sind.
9. Das Transportprotein nach einem der 1 bis 8, wobei das native Neurotoxin Neurotoxin Serotyp A ist und, vorzugsweise das Transportprotein an die synaptischen Vesikel Glykoproteine 2 (SV2), besonders bevorzugt SV2A, SV2B oder SV2C bindet.
10. Das Transportprotein nach 9, wobei mindestens eine Aminosäure in den Positionen Threonin 1195, Asparagin 1196, Glutamin 1199, Lysin 1204, Isoleucin 1205, Leucin 1206, Serin 1207, Leucin 1209, Aspartat 1213, Leucin 1217, Phenylalanin 1255, Asparagin 1256, Isoleucin 1258 und/oder Lysin 1260 der Botulinus Neurotoxin Serotyp A Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
11. Das Transportprotein nach 10, wobei mindestens eine Aminosäure in den Positionen Asparagin 1196, Glutamin 1199, Serin 1207, Phenylalanin 1255, Isoleucin 1258 und/oder Lysin 1260 posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
12. Das Transportprotein nach einem der 10 oder 11, wobei die Aminosäure Serin an Position 1207 durch Arginin oder Tyrosin ersetzt wird.
13. Das Transportprotein nach einem der 10 oder 11, wobei die Aminosäure Lysin an Position 1260 durch Glutamat ersetzt wird.
14. Das Transportprotein nach einem der 1 bis 8, wobei das Neurotoxin Botulinus Neurotoxin Serotyp B ist und, vorzugsweise das Transportprotein an Synaptotagmin,I oder II bindet.
15. Das Transportprotein nach 14, wobei mindestens eine Aminosäure in den Positionen Lysin 1113, Aspartat 1114, Serin 1116, Prolin 1117, Valin 1118, Threonin 1182, Tyrosin 1183, Phenylalanin 1186, Lysin 1188, Glutamat 1191, Lysin 1192, Leucin 1193, Phenylalanin 1194, Phenylalanin 1204, Phenylalanin 1243, Glutamat 1245, Lysin 1254, Aspartat 1255 und Tyrosin 1256 der Botulinus Neurotoxin Serotyp B Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
16. Das Transportprotein nach 15, wobei mindestens eine Aminosäure in den Positionen Valin 1118, Tyrosin 1183, Glutamat 1191, Lysin 1192, Glutamat 1245 und/oder Tyrosin 1256 der Botulinus Neurotoxin Serotyp B Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
17. Das Transportprotein nach 16, wobei die Aminosäure Tyrosin an Position 1183 durch Leucin substituiert ist.
18. Das Transportprotein nach 16, wobei die Aminosäure Glutamat an Position 1191 durch Leucin substituiert ist.
19. Das Transportprotein nach einem der 1 bis 8, wobei das Neurotoxin Botulinus Neurotoxin Serotyp G ist.
20. Das Transportprotein nach 19, wobei mindestens eine Aminosäure in den Positionen Phenylalanin 1121, Lysin 1123, Alanin 1124, Serin 1125, Methionin 1126, Valin 1190, Leucin 1191, Serin 1194, Glutamat 1196, Threonin 1199, Glutamin 1200, Leucin 1201, Phenylalanin 1202, Phenylalanin 1212, Phenylalanin 1248, Lysin 1250, Aspartat 1251 und Tyrosin 1262 der Botulinus Neurotoxin Serotyp G Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
21. Das Transportprotein nach 20, wobei mindestens eine Aminosäure in den Positionen Methionin 1126, Leucin 1191, Threonin 1199, Glutamin 1200, Lysin 1250 und Tyrosin 1262 der Botulinus Neurotoxin Serotyp G Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist.
22. Das Transportprotein nach 21, wobei die Aminosäure Tyrosin an Position 1262 durch Phenylalanin substituiert ist.
23. Eine Zusammensetzung, enthaltend ein Transportprotein nach einem der 1 bis 22 und wenigstens ein intervenierendes Molekül.
24. Die Zusammensetzung nach 23, wobei das intervenierende Molekül durch eine Peptid-Bindung, Ester-Bindung, Ether-Bindung, Sulfid-Bindung, Disulfid-Bindung oder Kohlenstoff-Kohlenstoff-Bindung kovalent an das Transportprotein gebunden ist.
25. Die Zusammensetzung nach 23 oder 24, wobei das intervenierende Molekül entweder ein kleines organisches Molekül, ein Peptid oder ein Protein ist.
26. Die Zusammensetzung nach 25, wobei das kleine organische Molekül ein Virustatikum, Zytostatikum, Antibiotikum oder ein Immunglobulin ist.
27. Die Zusammensetzung nach 25, wobei das Protein eine Protease ist.
28. Die Zusammensetzung nach 27, wobei die Protease eine oder mehrere leichte Ketten (LC) der Serotypen A, B, Cl, D, E, F und/oder G des *Clostridium botulinum* Neurotoxins umfasst.
29. Die Zusammensetzung nach 27, wobei die Protease ein proteolytisch aktives Fragment enthält, welches von der leichten Kette (LC) der Serotypen A, B, Cl, D, E, F und/oder G des *Clostridium botulinum* Neurotoxins abgeleitet ist und dadurch charakterisiert ist, dass es wenigstens 0,01 % der proteolytischen Aktivität der nativen Protease, vorzugsweise wenigstens 50 % aufweist.
30. Die Zusammensetzung nach 28 und 29, wobei die Protease bestimmte Substrate innerhalb des cholinergen Motoneurons spezifisch spaltet.
31. Die Zusammensetzung nach 30, wobei die Substrate ausgewählt sind aus Proteinen, die in der Freisetzung von Neurotransmittern in Nervenzellen involviert sind, und Proteinen, die zu katalytischen Reaktionen innerhalb der Nervenzelle fähig sind.
32. Die Zusammensetzung nach 28 und 29, wobei die Protease und das Transportprotein kovalent durch eine Aminosäuresequenz verbunden sind, die von einer Endopeptidase spezifisch erkannt und gespalten wird.
33. Die Zusammensetzung nach 32, wobei die Aminosäuresequenz die Sequenz CXXXZKTKSLVPRGSKBXXC umfasst, wobei X irgendeine Aminosäure ist und Z und B unabhängig voneinander ausgewählt sind aus Alanin, Valin, Serin, Threonin und Glycin.
34. Die Zusammensetzung nach 32, wobei nach Spaltung durch die Endopeptidase eine Disulfid-Brücke die Protease und das Transportprotein miteinander verbindet, was wiederum zur Entstehung eines aktiven Holotoxins führt.
35. Eine pharmazeutische Zusammensetzung, die das Transportprotein nach einem der 1 bis 22 oder die Zusammensetzung nach einem der 23 bis 34 enthält, sowie optional einen pharmazeutisch akzeptablen Träger, Verdünnungsmittel und/oder Additiv.
36. Verwendung der pharmazeutischen Zusammensetzung nach 35 zur Behandlung von Störungen und Erkrankungen, für welche eine Therapie mit Botulinus Neurotoxin indiziert ist.
37. Verwendung nach 36, wobei die Störung oder Erkrankung eine der folgenden ist: Hemifacialspasmus, spasmodischer Torticollis, Blepharospasmus, Spastizitäten, Dystonien, Migräne, Schmerzen, Erkrankungen der Hals- und Lendenwirbelsäule, Strabismus, Hypersalivation, Schnarchen, Wundheilung und depressive Erkrankungen.
38. Eine kosmetische Zusammensetzung, die das Transportprotein gemäß einem der
   1 bis 22 oder die Zusammensetzung nach einem der 23 bis 34 enthält, sowie optional einen kosmetisch akzeptablen Träger, Verdünnungsmittel und/oder Additiv.
39. Verwendung einer kosmetischen Zusammensetzung nach 38 für die Behandlung der kosmetischen Indikationen Hyperhydrose und Gesichtsfalten.
40. Ein Verfahren zur Herstellung eines Transportproteins nach den 1 bis 22 oder einer Zusammensetzung nach den 23 bis 34 durch Rekombination gemäß bekannter Verfahren.
41. Ein Verfahren zur Herstellung des Transportproteins nach 40, wobei das Gen des H_{C}-Fragments durch zwei Nukleinsäuren enthaltende Restriktionsendonukleaseschnittstellen flankiert wird, wobei die Restriktionsendonukleaseschnittstellen kompatibel sind mit jenen der anderen H_{C}-Fragmente aus *Clostridium botulinum* Neurotoxinen, um deren leichten modularen Austausch zu gestatten, während die Ähnlichkeit der Aminosäuresequenz erhalten bleibt.
42. Ein Verfahren zur Herstellung der Zusammensetzung nach 40, wobei das Gen der Protease durch zwei Nukleinsäuren enthaltende Restriktionsendonukleaseschnittstellen flankiert wird, wobei die Restriktionsendonukleaseschnittstellen kompatibel sind mit jenen der anderen Proteasedomänen aus *Clostridium botulinum* Neurotoxinen, um deren leichten modularen Austausch zu gestatten, während die Ähnlichkeit der Aminosäuresequenz erhalten bleibt.
43. Eine Wirtszelle, die einen rekombinanten Expressionsvektor enthält, wobei der Expressionsvektor ein Transportprotein gemäß den 1 bis 22 oder eine Zusammensetzung nach einem der 23 bis 34 kodiert.
44. Die Wirtszelle gemäß 43, wobei die Wirtszelle eine Zelle von *Escherichia coli,* insbesondere *E. coli* K12, *Saccharomyces cerevisiae, Pichia pastoris* oder *Bacillus megaterium* sein kann.
45. Ein Expressionsvektor, wobei der Vektor eine Nukleinsäure umfasst, die ein Transportprotein gemäß einem der 1 bis 22 oder eine Zusammensetzung nach einem der 23 bis 34 kodiert.

## Patentansprüche

1. Ein Transportprotein, erhältlich durch Modifizierung der schweren Kette des von *Clostridium botulinum* gebildeten Neurotoxins, wobei
(i) das Protein an Nervenzellen mit höherer oder niedrigerer Affinität bindet als das native Neurotoxin;
(ii) das Protein im Vergleich zu dem nativen Neurotoxin eine erhöhte oder verringerte Neurotoxizität aufweist; vorzugsweise wird die Neurotoxizität im Hemidiaphragma-Assay bestimmt; und/oder
(iii) das Protein im Vergleich zu dem nativen Neurotoxin eine geringere Affinität gegenüber neutralisierenden Antikörpern aufweist.

2. Das Transportprotein nach Anspruch 1, wobei:
i) der neutralisierende Antikörper die Bindung des nativen Neurotoxins an den Proteinrezeptor oder Gangliosidrezeptor verhindert und/oder die Aufnahme des Neurotoxins in die Nervenzelle verhindert; und/oder
ii) das Transportprotein durch Endozytose von den Zellen aufgenommen wird; und/oder
iii) das Protein spezifisch an die Plasmamembran assoziierte Moleküle, Transmembranproteine, synaptische Vesikelproteine, Proteine der Synaptotagmin-Familie oder der synaptischen Vesikel Glykoproteine 2 (SV2) und/oder Synaptotagmin I und/oder Synaptotagmin II (cholinerger Motoneurone) und/oder SV2A, SV2B oder SV2C bindet, bevorzugt humanes Synaptotagmin I und/oder humanes Synaptotagmip II und/oder humanes SV2A, SV2B oder SV2C; und/oder
iv) wobei das Transportprotein eine wenigstens 15 % höhere Affinität oder eine wenigstens 15 % niedrigere Affinität als das native Neurotoxin aufweist, vorzugsweise wenigstens 50 %, besonders bevorzugt wenigstens 80 %, insbesondere bevorzugt wenigstens 90 %; und/oder
v) wobei das H_{C}-Fragment des Transportproteins wenigstens eine Substitution und/oder Deletion und/oder Insertion und/oder Addition und/oder posttranslationale Modifikationen von Aminosäuren, die entweder natürlich vorkommen oder nicht natürlichen Ursprungs sind und die die Affinität gegenüber dem nativen Neurotoxin erhöhen oder erniedrigen, aufweist.

3. Das Transportprotein nach einem der Ansprüche 1 oder 2, wobei das Neurotoxin Botulinus Neurotoxin Serotyp A bis G ist;
wobei optional mindestens eine Aminosäure in den Aminosäurepositionen
867 bis 1296 des *Clostridium botulinum* Neurotoxin Serotyp A,
866 bis 1291 des *Clostridium botulinum* Neurotoxin Serotyp B,
864 bis 1291 bzw. 1280 des *Clostridium botulinum* Neurotoxin Serotyp Cl,
860 bis 1276 bzw. 1285 des *Clostridium botulinum* Neurotoxin Serotyp D,
843 bis 1251 bzw. 1252 des *Clostridium botulinum* bzw. *Clostridium butyricum* Neurotoxin Serotyp E,
861 bis 1274, 862 bis 1280 bzw. 1278 und 854 bis 1268 des *Clostridium botulinum* bzw. *Clostridium baratii* Neurotoxin Serotyp F,
861 bis 1297 des *Clostridium botulinum* Neurotoxin Serotyp G:
posttranslational modifiziert, und/oder addiert, und/oder deletiert, und/oder insertiert und/oder durch Aminosäuren substituiert ist, die entweder natürlich vorkommen oder nicht natürlichen Ursprungs sind.

4. Das Transportprotein nach einem der vorhergehenden Ansprüche, wobei:
i) das native Neurotoxin Neurotoxin Serotyp A ist und, vorzugsweise das Transportprotein an die synaptischen Vesikel Glykoproteine 2 (SV2), besonders bevorzugt SV2A, SV2B oder SV2C bindet; wobei optional
a) mindestens eine Aminosäure in den Positionen Threonin 1195, Asparagin 1196, Glutamin 1199, Lysin 1204, Isoleucin 1205, Leucin 1206, Serin 1207, Leucin 1209, Aspartat 1213, Leucin 1217, Phenylalanin 1255, Asparagin 1256, Isoleucin 1258 und/oder Lysin 1260 der Botulinus Neurotoxin Serotyp A Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist; und/oder
b) mindestens eine Aminosäure in den Positionen Asparagin 1196, Glutamin 1199, Serin 1207, Phenylalanin 1255, Isoleucin 1258 und/oder Lysin 1260 posttranslational modifiziert, und/oder addiert, und/oder deletiert, und/oder insertiert und/oder durch eine Aminosäure substituiert ist, die entweder natürlich vorkommt oder nicht natürlichen Ursprungs ist;
wobei vorzugsweise die Aminosäure Serin an Position 1207 durch Arginin oder Tyrosin ersetzt wird, oder die Aminosäure Lysin an Position 1260 durch Glutamat ersetzt wird; oder
ii) das Neurotoxin Botulinus Neurotoxin Serotyp B ist und vorzugsweise das Transportprotein an Synaptotagmin I oder II bindet, wobei optional:
a) mindestens eine Aminosäure in den Positionen Lysin 1113, Aspartat 1114, Serin 1116, Prolin 1117, Valin 1118, Threonin 1182, Tyrosin 1183, Phenylalanin 1186, Lysin 1188, Glutamat 1191, Lysin 1192, Leucin 1193, Phenylalanin 1194, Phenylalanin 1204, Phenylalanin 1243, Glutamat 1245, Lysin 1254, Aspartat 1255 und Tyrosin 1256 der Botulinus Neurotoxin Serotyp B Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist; und/oder
b) mindestens eine Aminosäure in den Positionen Valin 1118, Tyrosin 1183, Glutamat 1191, Lysin 1192, Glutamat 1245 und/oder Tyrosin 1256 der Botulinus Neurotoxin Serotyp B Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist;
wobei vorzugsweise die Aminosäure Tyrosin an Position 1183 durch Leucin substituiert ist, oder die Aminosäure Glutamat an Position 1191 durch Leucin substituiert ist; oder
iii) das Neurotoxin Botulinus Neurotoxin Serotyp G ist, wobei optional:
a) mindestens eine Aminosäure in den Positionen Phenylalanin 1121, Lysin 1123, Alanin 1124, Serin 1125, Methionin 1126, Valin 1190, Leucin 1191, Serin 1194, Glutamat 1196, Threonin 1199, Glutamin J 200, Leucin 1201, Phenylalanin 1202, Phenylalanin 1212, Phenylalanin 1248, Lysin 1250, Aspartat 1251 und Tyrosin 1262 der Botulinus Neurotoxin Serotyp G Proteinsequenzen posttranslational modifiziert, und/oder addie1i, und/oder deletiert, und/oder insertiert und/oder durch eine Aminosäure substituiert ist, die entweder natürlich vorkommt oder nicht natürlichen Ursprungs ist; und/oder
b) mindestens eine Aminosäure in den Positionen Methionin 1126, Leucin 1191, Threonin 1199, Glutamin 1200, Lysin 1250 und Tyrosin 1262 der Botulinus Neurotoxin Serotyp G Proteinsequenzen posttranslational modifiziert, und/oder addiert, und/oder deletiert, und/oder insertiert und/oder durch eine Aminosäure substituiert ist, die entweder natürlich vorkommt oder nicht natürlichen Ursprungs ist;
wobei vorzugsweise die Aminosäure Tyrosin an Position 1262 durch Phenylalanin substituiert ist.

5. Eine Zusammensetzung, enthaltend ein Transportprotein nach einem der Ansprüche 1 bis 4 und wenigstens ein intervenierendes Molekül, wobei optional das intervenierende Molekül durch eine Peptid-Bindung, Ester-Bindung, Ether-Bindung, Sulfid-Bindung, Disulfid- Bindung oder Kohlenstoff-Kohlenstoff-Bindung kovalent an das Transportprotein gebunden ist.

6. Die Zusammensetzung nach Anspruch 5, wobei das intervenierende Molekül entweder Folgendes ist:
i) ein kleines organisches Molekül, wobei optional das kleine organische Molekül ein Virostatikum, Zytostatikum, Antibiotikum oder ein Immunglobulin; oder
ii) ein Peptid oder ein Protein, wobei optional das Protein eine Protease ist.

7. Die Zusammensetzung nach Anspruch 6, wobei das Protein eine Protease ist, die:
i) eine oder mehrere leichte Ketten (LC) der Serotypen A, B, Cl, D, E, Fund/oder G des *Clostridium botulinum* Neurotoxins umfasst; oder
ii) ein proteolytisch aktives Fragment enthält, welches von der leichten Kette (LC) der Serotypen A, B, Cl, D, E, Fund/oder G des *Clostridium botulinum* Neurotoxins abgeleitet ist und dadurch charakterisiert ist, dass es wenigstens 0,01% der proteolytischen Aktivität der nativen Protease, vorzugsweise wenigstens 50 % aufweist.

8. Die Zusammensetzung nach Anspruch 7, wobei:
i) die Protease bestimmte Substrate innerhalb des cholinergen Motoneurons spezifisch spaltet, wobei optional die Substrate ausgewählt sind aus Proteinen, die in der Freisetzung von Neurotransmittern in Nervenzellen involviert sind, und Proteinen, die zu katalytischen Reaktionen innerhalb der Nervenzelle fähig sind; oder
ii) die Protease und das Transportprotein kovalent durch eine Aminosäuresequenz verbunden sind, die von einer Endopeptidase spezifisch erkannt und gespalten wird, wobei optional:
a) die Aminosäuresequenz die Sequenz CXXXZKTKSL VPRGSKBXXC umfasst, wobei X irgendeine Aminosäure ist und Z und B unabhängig voneinander ausgewählt sind aus Alanin, Valin, Serin, Threonin und Glycin; oder
b) nach Spaltung durch die Endopeptidase eine Disulfid-Brücke die Protease und das Transportprotein miteinander verbindet, was wiederum zur Entstehung eines aktiven Holotoxins führt.

9. Eine pharmazeutische Zusammensetzung, die das Transportprotein nach einem der Ansprüche 1 bis 4 oder die Zusammensetzung nach einem der Ansprüche 5 bis 8 enthält, sowie optional einen pharmazeutisch akzeptablen Träger, Verdünnungsmittel und/oder Additiv.

10. Pharmazeutische Zusammensetzung nach Anspruch 9 zur Verwendung in einem Verfahren der Behandlung von Störungen und Erkrankungen, für welche eine Therapie mit Botulinus Neurotoxin indiziert ist, wobei optional die Störung oder Erkrankung eine der folgenden ist: Hemifacialspasmus, spasmodischer Torticollis, Blepharospasmus, Spastizitäten, Dystonien, Migräne, Schmerzen, Erkrankungen der Hals- und Lendenwirbelsäule, Strabismus, Hypersalivation, Schnarchen, Wundheilung und depressive Erkrankungen.

11. Eine kosmetische Zusammensetzung, die das Transportprotein gemäß einem der Ansprüche 1 bis 4 oder die Zusammensetzung nach einem der Ansprüche 5 bis 8 enthält, sowie optional einen kosmetisch akzeptablen Träger, Verdünnungsmittel und/oder Additiv.

12. Verwendung einer kosmetischen Zusammensetzung nach Anspruch 11 für die Behandlung der kosmetischen Indikationen Hyperhydrose und Gesichtsfalten.

13. Ein Verfahren zur Herstellung eines Transportproteins nach den Ansprüchen 1 bis 4 oder einer Zusammensetzung nach den Ansprüchen 5 bis 8 durch Rekombination gemäß bekannter Verfahren, wobei optional:
i) das Gen des H_{C}-Fragments durch zwei Nukleinsäuren enthaltende Restriktionsendonukleaseschnittstellen flankiert wird, wobei die Restriktionsendonukleaseschnittstellen kompatibel sind mit Jenen der anderen H_{C}-Fragmente aus *Clostridium botulinum* Neurotoxinen, um deren leichten modularen Austausch zu gestatten, während die Ähnlichkeit der Aminosäuresequenz erhalten bleibt; oder
ii) das Gen der Protease durch zwei Nukleinsäuren enthaltende Restriktionsendonukleaseschnittstellen flankiert wird, wobei die Restriktionsendonukleaseschnittstellen kompatibel sind mit jenen der anderen Proteasedomänen aus *Clostridium botulinum* Neurotoxinen, um deren leichten modularen Austausch zu gestatten, während die Ähnlichkeit der Aminosäuresequenz erhalten bleibt.

14. Eine Wirtszelle, die einen rekombinanten Expressionsvektor enthält, wobei der Expressionsvektor ein Transportprotein gemäß den Ansprüchen 1 bis 4 oder eine Zusammensetzung nach einem der Ansprüche 5 bis 8 kodiert, wobei optional die Wirtszelle eine Zelle von *Escherichia coli,* insbesondere *E*. *coli* K12, *Saccharomyces cerevisiae, Pichia pastoris* oder *Bacillus megaterium* sein kann.

15. Ein Expressionsvektor, wobei der Vektor eine Nukleinsäure umfasst, die ein Transportprotein gemäß einem der Ansprüche 1 bis 4 oder eine Zusammensetzung nach einem der Ansprüche 5 bis 8 kodiert.
